# EUROPEAN PATENT SPECIFICATION

(11) **EP 2 233 099 B1**
(45) Date of publication and mention of the grant of the patent: **19.07.2017**
(21) Application number: 10157380.6
(22) Date of filing: 23.03.2010
(51) Int. Cl.: A61B 90/00

(54) **Computer-assisted system for guiding a surgical instrument during percutaneous diagnostic or therapeutic operations**
Rechnerunterstütztes System zum Führen eines chirurgischen Instruments während perkutaner Diagnostik oder therapeutischer Operationen
Système assistée par ordinateur pour guider un instrument chirurgical pendant diagnostic percutané ou opération thérapeutique

(30) Priority: 24.03.2009 EP 09425116
(43) Date of publication of application: 29.09.2010
(73) Proprietor: MASMEC S.p.A., 70026 Modugno (IT)
(72) Inventor: Vinci, Angelo Michele, 70026 Modugno (IT); Larizza, Pietro, 70026 Modugno (IT)
(74) Representative: Bergadano, Mirko

(56) References cited:
- WO-A1-2008/103383
- DE-U1- 29 623 941
- US-A- 5 394 457
- US-A1- 2003 179 856
- US-A1- 2004 092 815
- US-A1- 2006 104 707

## Description

### TECHNICAL FIELD OF THE INVENTION

The present invention relates to computer-assisted system for guiding a surgical instrument in a patient's body, in particular for assisting medical personnel during percutaneous diagnostic and/or therapeutic operations.

### STATE OF THE ART

As known, biopsies, radio-frequency thermal ablations and localizations are carried out today with the aid of a surgical instrument (needle or electrode-needle) capable of percutaneously reaching a zone concerned by a disease (often referred to as a "target zone"). The operation of introducing and subsequently reaching the target by the surgical instrument may be facilitated by guiding or navigation systems, of virtual type, based on images of the target and of the zones surrounding the target so as to plan and perform the percutaneous operation in a minimally invasive manner. For example, these images may be acquired in real time by means of an echography apparatus. However, for example during thoracic operations, the echography technique based on ultrasounds may not be used because the presence of air in the lungs does not allow to obtain intrathoracic images of sufficient quality. In general, the ultrasound echography technique may not be used in any situation in which a variation of acoustic impedance due to the presence of air may be envisaged. Furthermore, because the echography technique does not allow to acquire high resolution images, other methods have to be used to gather images useful for navigation.

The most common method adopted for acquiring thoracic images is computerized tomography (CT), which exploits ionizing radiations to obtain detailed images of specific body areas. Using computerized tomography does not allow however to acquire images in real time during the therapeutic operation because of the practical impossibility of handling the surgical instrument during the step of CT scanning of the subject on which the operation is being performed and because of the safety of the clinician who is performing the operation due to the harmfulness of ionizing radiations.

Known systems for image-assisted therapeutic operations are based on CT images acquired before the step of operating, for the purposes of a virtual-reality, three-dimensional reconstruction of the zone of the human body concerned by the surgical operation. A three-dimensional representation of the surgical instrument during the various steps of the operation is thus superimposed on such a three-dimensional reconstruction. It is apparent that for this purpose the surgical instrument should also be provided with appropriate sensors so that the three-dimensional representation thereof may be inserted in the three-dimensional reconstruction of the zone of the human body concerned by the surgical operation.

US 2003/179856 (A1) discloses an apparatus for determining a coordinate transformation for mixing an image of a first subject into an X-ray image of a second subject, said apparatus having an arrangement for fastening the apparatus to the second subject, x-ray transparent markers that can be acquired by an optical navigation system, and X-ray-positive marks, both positioned on an x-ray transparent frame. The positions and orientations of the markers that can be acquired with the navigation system and the positions and orientations of the X-ray-positive marks relative to one another are thus known, so a coordinate transformation can be determined between a coordinate system allocated to the navigation system and a coordinate system allocated to the X-ray image. Thus an image of the first subject that can be acquired with the navigation system can be mixed into an X-ray image of the second subject.

### OBJECT AND SUMMARY OF THE INVENTION

The Applicant found that the previously described CT method suffers from multiple critical aspects, the main of which has proven to be system calibration, i.e. the exact alignment between the three-dimensional reconstruction of the zone of the human body concerned by the surgical operation, which is based on CT images as mentioned, which images contain a two-dimensional representation of the organs of the human body in a given reference system due to their nature, and the three-dimensional representation of the surgical instrument which, as mentioned, is based on information provided by sensors, and thus expressed in the reference system of the sensors, which is inevitably different from that of CT images.

A further critical aspect of the previously described CT method is that the three-dimensional reconstruction of the zone of the human body concerned by the surgical operation is static because it is based on CT images taken in a precise instant prior to the operation. Therefore, the internal position of the organs which are actually subjected to movements, e.g. due to the expansion and contraction of the lungs when respiration, may not accurately be identified at every moment.

On the other hand, a critical aspect of general nature of computer-assisted guiding systems of a surgical instrument in a patient's body is that the surgical instrument is considered a rigid body not subject to deformations in use. Actually, on the contrary, a surgical instrument in the form of needle, or electrode-needle, bends in use, and the entity of the bending depends on the tissues that it crosses, consequently resulting in errors in reaching the target.

It is the object of the present invention to provide a computer-assisted guiding system for a surgical instrument in a patient's body which allows to mitigate one or more of the drawbacks of the known systems and methods and which further allows to improve the positioning and guiding of surgical instruments in percutaneous operations.

According to the present invention, a computer-assisted guiding system for a surgical instrument in a patient's body is provided as defined in the appended claims.

### BRIEF DESCRIPTION OF THE DRAWINGS

- Figure 1 diagrammatically shows a computer-assisted system for guiding a surgical instrument in a patient's body according to an embodiment of the present invention;
- Figure 2 is a partially exploded, perspective view of a patient marker device according to an embodiment of the present invention;
- Figure 3 is a side view of the patient marker device in figure 2;
- Figure 4 is a perspective view of a marker device for a surgical instrument according to an embodiment of the present invention.

### DETAILED DESCRIPTION OF PREFERRED EMBODIMENTS OF THE INVENTION

The present invention will now be described in detail with reference to the appended drawings to allow a person skilled in the art to implement it and use it. Various changes to the described embodiments will be immediately apparent to people skilled in the art, and the general principles described may be applied to other embodiments and applications without therefore departing from the scope of protection of the present invention, as defined in the appended claims. Therefore, the present invention should not be considered as being limited to the embodiments described and illustrated herein, but the broadest scope of protection in accordance with the principles and features described and claimed herein is to be conferred thereto.

The system for guiding a surgical instrument in a patient's body according to the present invention are particularly advantageous for providing assistance to a clinician during percutaneous diagnostic and therapeutic operations on a patient. Such a system and method is adapted to provide a volumetric (three-dimensional) reconstruction of the operation zone on the patient, which is displayed to the clinician during the operation. Furthermore, a three-dimensional model of the surgical instrument (e.g. biopsy needle, radio-frequency thermal ablation needle or another surgical instrument) used by the clinician is displayed to be integrated in the reconstructed volume of the operation zone.

The volumetric reconstruction of the patient's internal region concerned by the operation is made from a plurality of images acquired by means of computerized tomography (CT) of the patient. During the acquisition of these images, a marker device is arranged on the patient (in particular close to the operation region) and made integral with the patient him or herself (e.g. by using medical-purpose adhesive tape). The patient marker device is provided with first computerized marker elements which are opaque to tomography, adapted to define a first reference system with respect to the patient. Such a marker device is further provided with second marker elements which are transparent to computerized tomography. These second marker elements instead reflect infrared wavelengths generated, as described in greater detail below, by an appropriate infrared tracing sensor in a moment following the image acquisition by means of computerized tomography. The second marker elements are arranged on the patient marker device in a given, known position with respect to the first marker elements. By keeping the patient marker device in the same position as it takes during computerized tomography, a second reference system may be defined during the step of operating on the basis of the detected position of the second marker elements. By knowing the relative positions of the first and second marker elements, an association between the first and second reference systems can thus be created.

During operation, the surgical instrument is, in turn, coupled to a marker device thereof, similar to the patient marker device, but comprising third marker elements only, reflecting infrared wavelengths. When used in combination with the patient marker device, the instrument marker device allows to univocally trace the movement of the surgical instrument in the second reference system (correlated to the first reference system, as previously mentioned). Thereby, an image of the surgical instrument may be superimposed on the volumetric reconstruction made by means of computerized tomography, the position of which varies of the basis of the position variation of the instrument marker device.

By means of the surgical instrument marker up to six spatial coordinates may be acquired, three of which are Cartesian coordinates (axes X, Y, Z) and three are polar coordinates (angles α, β, γ), to univocally trace each movement of the surgical instrument handled by the clinician. Thereby, the trajectory and the penetration depth of the surgical instrument in the patient may be identified. Furthermore, the trajectory of the surgical instrument in depth inside the patient may be monitored by means of a guiding system with needle and sensorized cannula (provided with a sensor which detects the penetration depth inside the patient, for example), or by means of a telescopic variant thereof. The virtually reconstructed position of the surgical instrument inside the patient is thus correlated to the position of the internal organs reconstructed by means of computerized tomography.

An image of the patient portion concerned by the operation provided with a graduated scale is displayed to the clinician performing the operation, with the indication of the depth level reached by the surgical instrument (e.g. by showing the image of a surgical instrument superimposed on the image of the patient portion concerned by the operation).

With particular reference to needle or needle-cannula operation instruments or the like, in all cases of small diameter, the surgical instrument is subject to a considerable deformation when subjected to a penetration force inside the operation zone on the patient due to a plurality of factors, e.g. the nonuniformity of the tissues crossed during the insertion (in particular bone tissue). Possible deformations of the surgical instrument may be provided by making a mechanical model of the surgical instrument by using known modeling techniques, e.g. based on FEM - "Finite Element Modeling". According to the tissues encountered by the surgical instrument descending in depth into the patient (detected by means of computerized tomography), the mechanical movement of the surgical instrument allows to update the deformation it was subjected to in real time, on the basis of the parameters used during FEM modeling.

In addition to FEM modeling, in order to overcome the drawback due to the surgical instrument bending in use, the use of an additional needle-cannula may be included, which needle works as a rigid guide (or in all cases having a limited deformability). A variant of such a needle-cannula provides for its elongation/ contraction by means of a multi-stage telescopic conformation.

The needle-cannula (single stage or multi-stage) is oriented by virtue of the instrument marker device. In such a case, once the optimal insertion direction has been identified, it will work as a guide for the next insertion of the surgical instrument (biopsy needle, or radio-frequency thermal ablation needle, or localization needle).

By displaying the volumetric reconstruction of the patient's operation zone, the clinician may thus follow the trajectory of the surgical instrument traveling within the three-dimensionally reconstructed tissues.

A further limitation of the known systems is the impossibility of knowing the position of the internal organs in real time which, as shown, slightly varies according to the respiratory phase.

Such a limitation is overcome by the present invention performing multiple scans by means of computerized tomography, i.e. by acquiring a plurality (e.g. three) of sequences of CT images during the respective respiratory phases (e.g. aspiratory, mid-expiratory and total expiratory phases), so as to define respective volumetric spaces associated with these respiratory phases (by using multiple scans). Each respiratory phase is, for example, associated with a respective position taken by the patient marker device by detecting the position of the first marker elements. During the surgical operation, the position taken by the second marker elements (placed in a known position with respect to the first marker elements, as mentioned) is detected by means of the infrared tracing sensor. A patient's respiratory phase may thus be associated with the position detected by the second marker elements and the reconstruction of the operation zone associated with such a respiratory phase may be displayed to the clinician. Either alternatively or additionally, the patient's respiratory phases may be detected by means of convenient devices adapted for this purpose.

According to an alternative embodiment, a respiration detecting device (load cell) is placed on the patient's chest in order to take a respiration signal and identify a stable phase of the respiratory act on the basis thereof. Such a stable phase is stored and associated with an acquired tomography image, in particular to the tomography image acquired at such a stable phase. During the operation, the respiration signal is constantly monitored and the system according to the present invention provides the clinician with a go-ahead (e.g. by means of a sound signal) to perform the operation (e.g. a needle penetration) when the respiration signal reaches the same stable phase as the respiration signal acquired during the tomography scanning. The respiration detecting device may be a respiration detecting device specifically configured for this purpose (devices of this type are commercially available) or a marker device similar to the patient marker device, and may comprise marker elements thereof detectable by an infrared tracing sensor during the step of tomography scanning. In particular, as mentioned above, the infrared tracing signals of known type are capable of acquiring at least three Cartesian coordinates, and are thus capable of detecting one or more phases of the respiratory act.

The stable signal from the respiration sensor is stored during tomography scanning.

During the operation, the respiration signal is monitored to provide the clinician with an indication of when the respiration signal reaches the same state as the respiration signal stored during the step of scanning.

This condition makes the internal state of the tissues coherent between the image obtained by scanning and the current situation during the operation.

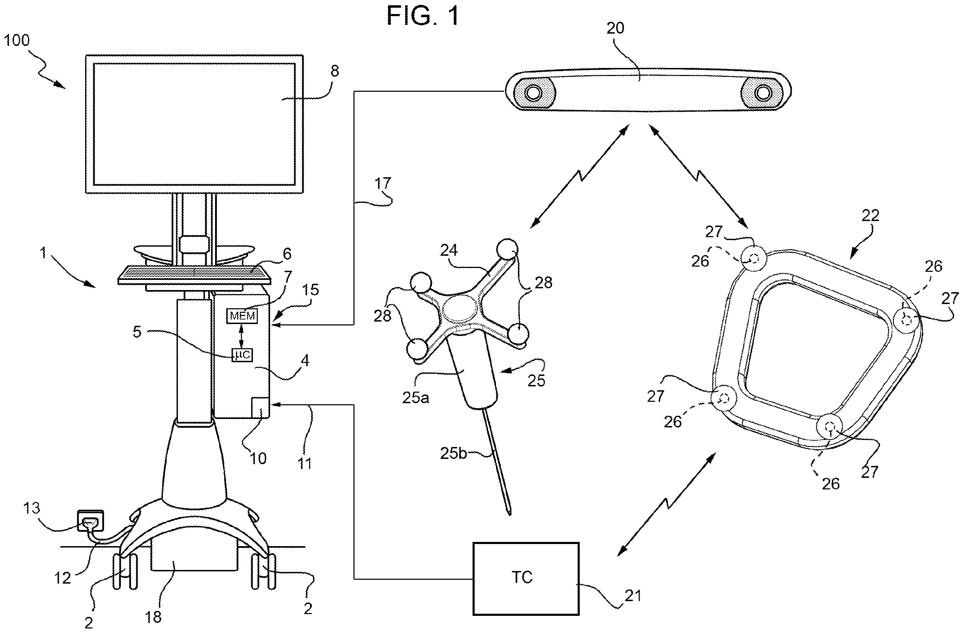

Figure 1 shows a guiding system 100 according to the present invention comprising an instrumental station 1; an infrared tracing sensor 20 of known type, configured to cooperate with the instrumental station 1; a patient marker 22 provided with first and second spherical marker elements 26, 27 concentric to each other, and configured to be arranged on a patient and to cooperate with a computerized tomography (CT) device 21 of known type and with the tracing sensor 20 (as described in greater detail below); and an instrument marker 24, provided with a plurality of third marker elements 28 and configured to be coupled to a surgical instrument 25 (in particular, a needle-electrode is shown in figure 1) and to cooperate with the tracing sensor 20 (as described in greater detail below). In particular, the surgical instrument 25 comprises a proximal, graspable portion 25a and an operative, distal portion 25b; the instrument marker 24 is coupled to the proximal portion 25a, leaving the distal portion 25b free. Here, it is noted that the relative dimensions of the elements shown in figure 1 are not proportional to one another, for a greater clarity of the drawing.

The instrumental station 1, movable on wheels 2, comprises a processing unit 4, e.g. a processor of known type provided with a microcontroller 5 and a memory 7, connected to each other; a data input user interface 6, e.g. including a keyboard and a mouse; a displaying interface 8, e.g. a high resolution monitor; a network interface 10, configured to support a connection to a private and/or public network 11 (e.g. an Ethernet type network, diagrammatically shown in figure 1 with a two-way arrow), in particular to request and/or receive image data related to a patient from a computerized tomography apparatus 21, provided with a patient marker 22 arranged in contact with the patient close to the region concerned by the operation, subjected to CT scanning; a power connection 12, configured to supply electricity to the instrumental station 1 by means of a wall-mounted power socket 13; a tracing input 15, configured to support a connection 17 (of either the wireless or wired type) between the instrumental station 1 and the tracing sensor 20; and an energy storing unit 18, e.g. a battery, connected to the wall-mounted socket 13 by means of the power connection 12 and configured to temporarily supply power to the instrumental station 1 in case of interruption of the power supplied by the wall-mounted power socket 13.

According to the illustrated embodiment, the processing unit 4 is a personal computer (PC), comprising an external protective body accommodated on a shelf of the instrumental station 1 and integral with the instrumental station 1 during a possible movement of the latter on the wheels 2.

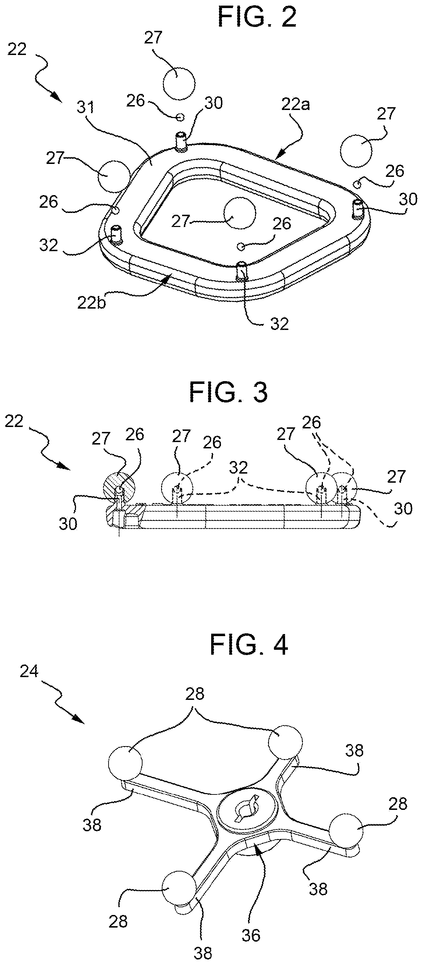

In order to acquire images inside the patient's body to make a volumetric reconstruction of the operation zone, the patient marker 22 is arranged in contact with the patient so as to be integral therewith (e.g. by means of adhesive tape for medical use) in a patient region (externally) proximal to the operation region. Therefore, the patient will simply need to behave as a rigid body only in the zone concerned by the operation, while the other body parts are free to move. The patient marker 22 is indeed sufficiently small to allow the patient not to be forced in a given position. The patient marker 22 is described in greater detail with reference to figures 2 and 3.

The patient provided with the patient marker 22 is thus subjected to computerized tomography by means of the computerized tomography apparatus 21. Alternatively, in order to acquire images inside the patient a different apparatus may be used, e.g. a magnetic resonance system or apparatus (not shown), providing that it may provide two-dimensional or three-dimensional images of the patient's internal organs and of the first marker elements 26 of the patient marker 22. The first marker elements 26 may be made of a plurality of materials, providing that these materials are opaque for the image acquisition system and method used, and thus may be identified in the acquired images (by CT, magnetic resonance, etc.). According to the embodiment shown in figure 1, the computerized tomography apparatus 21 is connected to the processing unit 4 to send the acquired images to the processing unit 4. The two-dimensional images acquired by the computerized tomography apparatus 21 are processed by the processing unit 4to obtain a three-dimensional representation, by means of known techniques widely used for this purpose.

Furthermore, the acquisition of images by means of computerized tomography conveniently comprises the acquisition of images during a plurality of respiratory phases of the patient, e.g. during aspiratory, mid-expiratory and total expiratory phases. A three-dimensional reconstruction of the internal operation region of the patient is therefore made for each respiratory phase so as to define respective volumetric spaces associated with these respiratory phases. In particular, the variation of the internal arrangement of organs and tissues may be identified during the various respiratory phases.

Figures 2 and 3 show an exploded perspective view and a side view of the patient marker 22, respectively. According to the embodiment shown, the patient marker 22 comprises a body 31, e.g. made of plastic material or more generally of a material transparent to the image acquisition system used. The body 31 is substantially trapezoidal in shape. Other shapes are possible, e.g. cross-shaped or X-shaped. The body 31 has a longer side 22a, the ends of which are provided with respective first coupling elements 30, and a smaller side 22b the ends of which are provided with respective second coupling elements 32. The first and second coupling elements 30 and 32 are configured to be coupled with first and second marker elements 26 and 27. In particular, both the first and second marker elements 26, 27 are spherical in shape. As shown in greater detail in figure 3, the second marker elements 27 are provided with an inner housing such as to contain the first marker elements 26 so that they are concentric when coupled and seen from above. The second marker elements 27, and thus the first marker elements 26 accommodated therein, are integrally coupled in use to respective first and second coupling elements 30, 32. However, it is worth noting that for hygienic reasons, the first and second marker elements 26, 27 may be replaced after each use. Advantageously, the second marker elements 27 accommodating the first marker elements 26 and the first and second coupling elements 30, 32 may be snap-coupled. The first marker elements 26 have a high HU (Hounsfield Unit) value, so as to be opaque to CT; on the contrary, the second marker elements 27 have a very low HU value, so as to be transparent to CT. However, the second marker elements 27 are of the infrared reflecting type in order to be detected by the tracing sensor 20.

The first and second marker elements 26, 27 may be accommodated with respect to each other in a different manner from that shown in figure 2 and 3, e.g. they may not be concentric, but arranged according to a predetermined, freely chosen relationship. Regardless of the chosen reciprocal arrangement, the first and second marker elements 26, being respectively adapted to be identified by means of the computerized tomography apparatus 21 and the tracing sensor 20, allow to define the respective coordinate systems for the images acquired by the computerized tomography apparatus 21 and for the images acquired by the tracing sensor 20. The coordinate systems may thus be correlated knowing the relative arrangement of the first and second marker elements 26, 27.

Figure 4 shows an embodiment of the instrument marker 24, particularly usable with a surgical, thermal ablation instrument. The marker instrument 24 comprises a central body 36 having a substantially circular shape and variable diameter, to be adapted to operating instruments 25 of different type and different size, and a plurality of arms 38 (four arms 38 are shown in the embodiment shown in figure 4, but more or fewer than four arms may be present), extending as spokes from the central body 36, substantially forming a cross. Each arm 38 accommodates a respective third, spherical-shaped marker element 28 at an end portion thereof. Each marker element 28 is made of infrared reflecting materials so as to be identified by the tracing sensor 20. It is apparent that if a tracing sensor 20 of the non-infrared type is used, each third marker element 28 will be conveniently made of a material identifiable by the particular type of tracing sensor 20 used.

The arrangement of the third marker elements 28 with respect to one another may be used by the processing unit 4 to univocally identify a particular type of instrument marker 24. Thereby, by providing a plurality of instrument markers 24, each of them having its arrangement of third marker elements 28 different from the arrangement of the third marker elements 28 of other instrument markers 24, a marker instrument 24 may be univocally associated with a particular type of surgical instrument 25. The processing unit 4, by means of the tracing sensor 20, may thus automatically recognize which surgical instrument 25 is being used by identifying a given arrangement of third marker elements 28. In this case, particular care should be adopted by the clinician in coupling each surgical instrument 25 with the correct instrument marker 24. The association between the arrangement of third marker elements 28 and surgical instrument 25 may be obviously manually modified by intervening on the processing unit 4.

The third marker elements 28 are further arranged with respect to one another so that both a rotation and a displacement of the instrument marker 24 (and thus of the surgical instrument 25 with which the instrument marker 24 is integral) are immediately detectable. With this regard, the applicant observed that the arrangement of the third marker elements 28 each at a respective end of a cross allows good results to be obtained. Further sensors may be provided on the surgical instrument in order to detect the penetration depth of the surgical instrument 25 in use. For example, as previously mentioned, such a depth may be detected by monitoring the Cartesian coordinates X, Y, Z and the polar coordinates α, β, γ of the surgical instrument 25 by monitoring the Cartesian coordinates X, Y, Z and the polar coordinates α, β, γ of the second marker elements 28. A guide with needle and sensorized cannula of known type (i.e. provided with a sensor which detect the penetration depth thereof inside the patient) or a telescopic variant thereof may be used either additionally or alternatively. The reached depth is thus shown to the clinician performing the operation by means of the displaying interface 8 using a graduated scale, for example.

Also with reference to figures 1-4, at the end of the image acquisition step by the computerized tomography apparatus 21, the patient is arranged so that the patient marker 22 is seen by the tracing sensor 20. The tracing sensor 20 detects and sends the spatial arrangement detected by the second marker elements 27 to the processing unit 4, according to a reference system thereof. Furthermore, as mentioned, since the surgical instrument 25 is provided in use with the instrument marker 24, the spatial arrangement of the third marker elements 28 is also detected by the tracing sensor 20 according to reference system thereof and sent to the processing unit 4. By knowing the arrangement of the first marker elements 26 obtained from the images acquired by the computerised tomography apparatus 21 (in a reference system of the computerised tomography apparatus itself), knowing the relative arrangement of the second marker elements 27 detected by the tracing sensor 20 (in a reference system of the tracing sensor 20 itself), and knowing the relative arrangement (detected in real time in the reference system of the tracing sensor 20 itself) of the third marker elements 28 arranged on the instrument marker 24 as compared to the arrangement of the second marker elements 27, the processing unit 4 may univocally identify the position of the third marker elements 28 (and thus of the surgical instrument 25) with respect to the first marker elements 26. Thereby, the reference systems of the computerised tomography apparatus 21 and of the tracing sensor 20 are related to one another, so that the virtual reciprocal arrangement of the first and second marker elements 26, 27 in the tracing sensor reference system 20 corresponds to the real reciprocal arrangement thereof. The position of the third marker elements 28 acquired in the reference system of the tracing marker 30 may therefore be determined in the reference system of the computerized tomography apparatus 21 on the basis of this relation.

The arrangement of the surgical instrument 25, univocally identified by the marker instrument 24, may thus be represented with respect to the images of the patient acquired by means of computerized tomography.

Both first and second marker elements 26, 26 and the third marker elements 28 are fixed in a given geometrical arrangement onto the patient marker 22 and on the instrument marker 24, respectively, so that the determination of the position of the patient marker 22 and of the instrument marker 24 is univocally defined in a three-dimensional space.

During the steps of operating on the patient, the patient marker 22 and the instrument marker 24 should be kept visible for the tracing sensor 20, so that the position of the surgical instrument 25 with respect to the operation region on the patient is always identifiable.

During the steps of operation, a three-dimensional model of the patient zone concerned by the operation, i.e. displaying the internal organs and tissues, is shown on the displaying interface 8. Such a three-dimensional model is generated, as mentioned, by the processing unit 4. By monitoring the patient's respiratory phases during the operation, the image displayed on the displaying interface 8 is constantly updated in real time with the corresponding image of the current respiratory phase. For this purpose, as mentioned above, a respiration detecting device (load cell) is arranged on the patient's chest in order to take a respiration signal. During the tomography scanning, the stable signal from the respiration sensor in a plurality of respiratory phases (e.g. maximum aspiratory phase, maximum expiratory phase, intermediate state) is stored. During the operation, the respiration signal is constantly monitored to provide the clinician with an indication of when the respiration signal reaches the same state as the respiration signal stored during the step of tomography scanning (coinciding to the maximum aspiratory phase, the maximum expiratory phase, the intermediate state).

Thereby, the interior position of the tissues may be made coherent with the image obtained by computerized tomography scanning and with the current position during the operation. By observing the displaying interface 8 (and/or being warned by a sound signal), the clinician checks when the respiration signals reaches the same state as the respiration signal stored during the step of scanning to proceed with the steps of the operation.

Alternatively, during the tomography scanning, the stable signal from the respiration sensor in a single respiratory phase is stored (e.g. only one of the maximum aspiratory phase, the maximum expiratory phase and the intermediate state). During the operation, the respiration signal is constantly monitored to provide the clinician with an indication of when the respiration signal reaches the same state as the respiration signal stored during the step of tomography scanning (coinciding to one of the maximum aspiratory phase, the maximum expiratory phase and the intermediate state). Such an indication may be provided to the clinician, for example, by means of a sound signal. The clinician may thus proceed with the operation (e.g. penetrating a needle) only when it is guided by the sound signal because only in such a state there is the real inner state of the tissues coherent with the image obtained by the tomography scanning.

Furthermore, a three-dimensional model of the surgical instrument used is superimposed on the three-dimensional model of the patient's zone concerned by the operation on the displaying interface 8. The type of surgical instrument 25 to be displayed may be chosen by the operating clinician from a plurality of possible models, previously made and stored in the memory 7 of the processing unit 4. Alternatively, the type of surgical instrument 25 may be automatically chosen by the processing unit 4 according to the arrangement of the third marker devices 28 of the instrument marker 24 (as previously described).

The clinician handling the surgical instrument 25 provided with the instrument marker 24 is guided during the whole operation by the images displayed on the displaying interface 8. The trajectory of the surgical instrument 25 is calculated with the support of artificial intelligence algorithms so that the whole concerned area is treated with the minimum number of penetrations, thus ensuring a total coverage and absence of collisions with vital organs and/or impediments. These organs are identified by means of the three-dimensional model shown on the displaying interface 8. The position of the surgical instrument 25 is inserted in the three-dimensional model by virtue of the measurement made by the tracing sensor 20 on the spatial coordinates of the instrument marker 24. In practice, up to three Cartesian coordinates (X, Y, Z) and up to three polar coordinates (α, β, γ) may be acquired. As a result, the real movements of the surgical instrument 25 are transferred in real time by means of the connection 17 to the processing unit 4 for obtaining an updated representation of the state of the operation on the displaying interface 8. Alternatively, in a manner similar to the above description, the trajectory of the surgical instrument 25 may be simulated *a priori* in order to establish the optimal trajectory once the target to be treated has been identified. Once the optimal trajectory has been determined, the penetration is carried out by the clinician handling the surgical instrument 25 provided with the instrument marker 24.

In particular, in the case of thermal ablation, the reconstruction of the trajectory of the surgical instrument 25 is calculated with the support of artificial intelligence algorithms, as mentioned, e.g. using genetic algorithms, and has the advantage of allowing the optimal trajectories to be established for the operation. The optimization criteria used is, in the particular case of thermal ablation, based on minimizing the number of insertions and maximizing the covered volume.

Furthermore, the use of mechanical models of operating instruments by means of modeling techniques, such as, for example, Finite Element Modeling (FEM) allows to evaluate possible deformations of the surgical instrument in use to update the displayed trajectory of the surgical instrument in real time, on the basis of the calculation of deformation it undergoes under the bias of the force of gravity. This function is also advantageous if a system comprises a rigid cannula for guiding the surgical instrument.

The advantages of the present system and method thereof are the following.

The registration or calibration of the images in the various reference systems (CT and infrared) is simply and automatically carried out by comparing the respective reference systems on the basis of the detected arrangement of CT opaque and infrared reflecting marker elements arranged on the patient marker. Thereby, the system is capable of self-calibrating thus making the various reference systems consistent.

Furthermore, the problem of the possible deformation of the surgical instrument in use is solved by using mathematical models (e.g. made by means of known FEM techniques) to simulate the behavior of the surgical instrument in use.

Finally, the risks of injury to internal organs during the operating step are considerably reduced by monitoring the patient's respiratory activity and displaying a reconstruction of the patient's internal organs varying according to the current respiratory stage.

It is finally apparent that changes and variations may be made to the system and method described and illustrated herein, without therefore departing from the scope of protection of the present invention, as defined in the appended claims.

## Claims

1. A computer-assisted system (100) for guiding a surgical instrument (25) in the body of a patient comprising:
• a patient marker device (22) configured to be arranged so as to be integral with a patient's body region to be treated by means of said surgical instrument (25), and including a tomography-transparent supporting frame (31) fitted with tomography-opaque first marker elements (26) and infrared-reflective, tomography-transparent second marker elements (27), said first and second marker elements (26, 27) being arranged on the supporting frame (31) in pairs, with the first marker elements (26) arranged within the paired second marker elements (27), and said first and second marker elements (26, 27) having a given real reciprocal arrangement;
• an instrument marker device (24) configured to be coupled to said surgical instrument (25) and including infrared-reflective third marker elements (28) ;
• an infrared locating sensor (20) configured to locate said infrared-reflective second and third marker elements (27, 28) in a first reference system; and
• a processing unit (4) connected to the locating sensor (20) and configured to:
- acquire at least one tomography image of the patient's body region where the patient marker device (22) is arranged, said tomography image including a representation of said tomography-opaque first marker elements (26) in a second reference system different from the first reference system;
- acquire the position of said infrared-reflective second and third marker elements (27, 28) in the first reference system provided by the infrared locating sensor (20);
- reciprocally correlate the first and second reference systems so that the virtual reciprocal arrangement of the first and second marker elements (26, 27) in the second reference system corresponds to the real reciprocal arrangement thereof; and
- determine the position of said infrared-reflective third marker elements (28) in the second reference system on the basis of the correlation between the first and second reference systems.

2. A system according to claim 1, further comprising:
• a displaying unit (8) connected to the processing unit (4);
wherein said processing unit (4) further comprises a memory (7) configured to store at least one numerical model of the surgical instrument (25);
and wherein said processing unit (4) is further configured to cause said displaying unit (8) to display:
- a three-dimensional representation of said patient's body region generated on the basis of said acquired tomography image; and
- a three-dimensional representation at least of the operating portion (25b) of said surgical instrument (25), graphically superimposed on said three-dimensional representation of said patient's body region, on the basis of the position of said third marker elements (28) in the second reference system and of the numerical model of the surgical instrument (25) stored in said memory (7).

3. A system according to claim 2, wherein said tomography image of the patient's body region relates to a given respiratory phase of the patient;
and wherein said processing unit (4) is further configured to:
- determine the patient's current respiratory phase; and
- cause the correspondence of the patient's current respiratory phase to that of said tomography image to be signalled.

4. A system according to claim 2, wherein said processing unit (4) is further configured to:
- acquire different tomography images of the patient's body region in which the patient marker device (22) is arranged and related to different respiratory phases of the patient, and
- cause said displaying unit (8) to displaya dynamic three-dimensional representation of said patient's body region during various respiratory phases of the patient, generated on the basis of the corresponding tomography images acquired.

5. A system according to any one of the preceding claims, wherein said memory (7) of said processing unit (4) is configured to store numerical models of different operating instruments (25);
wherein said system (100) comprises different instrument marker devices (24) provided with corresponding third marker elements (28) having different arrangements which may be associated with different surgical instruments (25) to allow the identification thereof;
and wherein said processing unit (4) is further configured to:
- identifying the surgical instrument (25) used on the basis of the arrangement of the third marker elements (28) associated therewith;
- cause said displaying unit (8) to display the corresponding three-dimensional representation on the basis of the corresponding numerical model stored.

## Patentansprüche

1. Computergestütztes System (100) zum Führen eines chirurgischen Instruments (25) im Körper eines Patienten, mit:
einer Patientenmarkierungseinrichtung (22), die dafür konfiguriert ist, derart angeordnet zu werden, dass sie mit dem durch das chirurgische Instrument (25) zu behandelnden Körperbereich eines Patienten integral ist, und einen tomografie-transparenten Trägerrahmen (31) aufweist, auf dem tomografie-opake erste Markierungselemente (26) und Infrarotlicht reflektierende tomografietransparente zweite Markierungselemente (27) angepasst sind, wobei die ersten und die zweiten Markierungselemente (26, 27) auf dem Trägerrahmen (31) paarweise angeordnet sind, wobei die ersten Markierungselemente (26) innerhalb des Paars zweiter Markierungselemente (27) angeordnet sind, und wobei die ersten und zweiten Markierungselemente (26, 27) eine vorgegebene reale reziproke Anordnung haben;
einer Instrumentenmarkierungseinrichtung (24), die dafür konfiguriert ist, mit dem chirurgischen Instrument (25) verbunden zu werden und Infrarotlicht reflektierende dritte Markierungselemente (28) aufweist;
einem Infrarotlichtlokalisierungssensor (20), der dafür konfiguriert ist, die Infrarotlicht reflektierenden zweiten und dritten Markierungselemente (27, 28) in einem ersten Bezugssystem zu lokalisieren; und
einer mit dem Lokalisierungssensor (20) verbundenen Verarbeitungseinheit (4), die dafür konfiguriert ist:
mindestens ein Tomografiebild des Körperbereichs des Patienten, wo die Patientenmarkierungseinrichtung (22) angeordnet ist, zu erfassen, wobei das Tomografiebild eine Darstellung der tomografie-opaken ersten Markierungselemente (26) in einem vom ersten Bezugssystem verschiedenen zweiten Bezugssystem aufweist;
die Position der Infrarotlicht reflektierenden zweiten und dritten Markierungselemente (27, 28) in dem durch den Infrarotlichtlokalisierungssensor (20) bereitgestellten ersten Bezugssystem zu erfassen;
das erste und das zweite Bezugssystem reziprok zu korrelieren, so dass die virtuelle reziproke Anordnung der ersten und zweiten Markierungselemente (26, 27) im zweiten Bezugssystem ihrer realen reziproken Anordnung entspricht; und die Position der Infrarotlicht reflektierenden dritten Markierungselemente (28) im zweiten Bezugssystem auf der Basis der Korrelation zwischen dem ersten und dem zweiten Bezugssystem zu bestimmen.

2. System nach Anspruch 1, ferner mit:
einer mit der Verarbeitungseinheit (4) verbundenen Displayeinheit (8),
wobei die Verarbeitungseinheit (4) ferner einen Speicher (7) aufweist, der dafür konfiguriert ist, mindestens ein numerisches Modell des chirurgischen Instruments (25) zu speichern,
und wobei die Verarbeitungseinheit (4) ferner dafür konfiguriert ist, zu veranlassen, dass die Displayeinheit (8) darstellt:
eine dreidimensionale Darstellung des Körperbereichs des Patienten, die auf der Basis des erfassten Tomografiebildes erzeugt wurde; und
eine dreidimensionale Darstellung mindestens des Arbeitsbereichs (25b) des chirurgischen Instruments (25), die auf der dreidimensionalen Darstellung des Körperbereichs des Patienten dreidimensional überlagert ist, auf der Basis der Position der dritten Markierungselemente (28) im zweiten Bezugssystem und des im Speicher (7) gespeicherten numerischen Modells des chirurgischen Instruments (25).

3. System nach Anspruch 2, wobei das Tomografiebild des Körperbereichs des Patienten mit einer vorgegebenen Atemphase des Patienten in Beziehung steht,
und wobei die Verarbeitungseinheit (4) ferner dafür konfiguriert ist:
die aktuelle Atemphase des Patienten zu bestimmen; und
die Zuordnung der aktuellen Atemphase des Patienten zu derjenigen des zu signalisierenden Tomografiebildes zu veranlassen.

4. System nach Anspruch 2, wobei die Verarbeitungseinheit (4) ferner dafür konfiguriert ist:
verschiedene Tomografiebilder des Körperbereichs des Patienten zu erfassen, in denen die Patientenmarkierungseinrichtung (22) angeordnet ist, und die mit verschiedenen Atemphasen des Patienten in Beziehung stehen; und
zu veranlassen, dass die Displayeinheit (8) eine dynamische dreidimensionale Darstellung des Körperbereichs des Patienten während verschiedenen Atemphasen des Patienten darstellt, die auf der Basis der entsprechenden erfassten Tomografiebilder erzeugt wurde.

5. System nach einem der vorangehenden Ansprüche, wobei der Speicher (7) der Verarbeitungseinheit (4) dafür konfiguriert ist, numerische Modelle verschiedener chirurgischer Instrumente (25) zu speichern,
wobei das System (100) verschiedene Instrumentmarkierungseinrichtungen (24) aufweist, die entsprechende dritte Markierungselemente (28) mit verschiedenen Anordnungen aufweisen, die den verschiedenen chirurgischen Instrumenten (25) zugewiesen sein können, um ihre Identifizierung zu ermöglichen,
und wobei die Verarbeitungseinheit (4) ferner dafür konfiguriert ist,
das verwendete chirurgische Instrument (25) auf der Basis der Anordnung der ihm zugeordneten dritten Markierungselemente (28) zu identifizieren; und
zu veranlassen, dass die Displayeinheit (8) die entsprechende dreidimensionale Darstellung auf der Basis des entsprechenden gespeicherten numerischen Modells darstellt.

## Revendications

1. Système assisté par ordinateur (100) pour guider un instrument chirurgical (25) dans le corps d'un patient comportant :
• un dispositif marqueur de patient (22) configuré pour être intégré à la région à traiter du corps d'un patient par des moyens dudit instrument chirurgical (25), et comprenant un cadre support transparent de tomographie (31) doté de premiers éléments marqueurs opaques de tomographie (26) et de deuxièmes éléments marqueurs transparents de tomographie (27), réfléchissants dans le domaine des infrarouges, lesdits premiers et deuxièmes éléments marqueurs (26, 27) étant agencés par paires sur le cadre support (31) tels que les premiers éléments marqueurs (26) sont couplés avec les deuxièmes éléments marqueurs (27), et les premiers et deuxièmes éléments marqueurs (26, 27) ayant un agencement réciproque réel donné;
• un dispositif marqueur d'instrument (24) configuré pour être couplé audit instrument chirurgical (25) et comprenant des troisièmes éléments marqueurs (28) réfléchissants dans le domaine des infrarouges;
• un capteur de position par infrarouge (20) configuré pour localiser lesdits deuxième et troisièmes éléments marqueurs à réflexion par infrarouge (27, 28) dans un premier système de référence; et
• une unité de traitement (4) connectée au capteur de position (20) et configurée pour:
- acquérir au moins une image tomographique de la région du corps du patient où est disposé le dispositif marqueur du patient (22), ladite image tomographique comprenant une représentation desdits premiers éléments marqueurs opaques de tomographie (26) dans un second système de référence différent du premier système de référence ;
- acquérir la position desdits deuxièmes et troisièmes éléments marqueurs à réflexion par infrarouge (27, 28) dans le premier système de référence fourni par le capteur de position (20);
- corréler réciproquement les premier et second systèmes de référence de sorte que l'agencement virtuel réciproque des premiers et deuxièmes éléments marqueurs (26, 27) dans le second système de référence corresponde à l'agencement réel réciproque de ceux-ci; et
- déterminer la position desdits troisièmes éléments marqueurs à réflexion par infrarouge (28) dans le second système de référence sur la base de la corrélation entre les premier et second systèmes de référence.

2. Système selon la revendication 1, comportant en outre :
• une unité d'affichage (8) connectée à l'unité de traitement (4);
dans lequel ladite unité de traitement (4) comprend en outre une mémoire (7) configurée pour stocker au moins un modèle numérique de l'instrument chirurgical (25);
et dans lequel ladite unité de traitement (4) est en outre configurée pour afficher sur ladite unité d'affichage (8) :
- une représentation tridimensionnelle de la région du corps du patient générée sur la base de ladite image tomographique acquise; et
- une représentation tridimensionnelle au moins de la partie opérationnelle (25b) dudit instrument chirurgical (25), superposée graphiquement sur ladite représentation tridimensionnelle de ladite région du corps du patient, sur la base de la position desdits troisièmes éléments marqueurs (28) dans le second système de référence et du modèle numérique de l'instrument chirurgical (25) mémorisé dans ladite mémoire (7).

3. Système selon la revendication 2, dans lequel ladite image tomographique de la région du corps du patient se rapporte à une phase respiratoire donnée du patient;
et dans lequel ladite unité de traitement (4) est en outre configurée pour:
- déterminer la phase respiratoire actuelle du patient; et
- engendrer la correspondance de la phase respiratoire actuelle du patient pour que l'image tomographique soit signalée.

4. Système selon la revendication 2, dans lequel ladite unité de traitement (4) est en outre configurée pour:
- acquérir différentes images tomographiques de la région du corps du patient dans laquelle le dispositif marqueur du patient (22) est agencé et lié à différentes phases respiratoires du patient, et
- afficher sur ladite unité d'affichage (8) une représentation tridimensionnelle dynamique de la région du corps dudit patient pendant diverses phases respiratoires du patient, générée sur la base des images tomographiques correspondantes acquises.

5. Système selon l'une quelconque des revendications précédentes, dans lequel ladite mémoire (7) de ladite unité de traitement (4) est configurée pour stocker des modèles numériques d'instruments chirurgicaux différents (25);
dans lequel ledit système (100) comprend différents dispositifs marqueurs d'instrument (24) dotés de troisièmes éléments marqueurs correspondants (28) ayant des agencements différents qui peuvent être associés à différents instruments chirurgicaux (25) pour permettre leur identification;
et dans lequel ladite unité de traitement (4) est en outre configurée pour :
- identifier l'instrument chirurgical (25) utilisé sur la base de l'agencement des troisièmes éléments marqueurs (28) qui lui sont associés;
- afficher sur ladite unité d'affichage (8) la représentation tridimensionnelle correspondante sur la base du modèle numérique correspondant enregistré.
